# EUROPEAN PATENT SPECIFICATION

(11) **EP 2 838 613 B1**
(45) Date of publication and mention of the grant of the patent: **07.06.2017**
(21) Application number: 13719763.8
(22) Date of filing: 15.04.2013
(51) Int. Cl.: A61N 5/06, A61B 18/20

(54) **MEDICAL SYSTEM FOR TREATING DEEP SURFACE TARGETS**
MEDIZINISCHES SYSTEM ZUR BEHANDLUNG VON TIEFENGEWEBEZIELEN
SYSTÈME MÉDICAL DE TRAITEMENT DE CIBLES DE SURFACE PROFONDES

(30) Priority: 18.04.2012 EP 12164678; 23.05.2012 US 201261650638 P; 29.06.2012 EP 12174266
(43) Date of publication of application: 25.02.2015
(73) Proprietor: Fatemi, Afschin, Dr., 40474 Duesseldorf (DE)
(72) Inventor: Fatemi, Afschin, Dr., 40474 Duesseldorf (DE)
(74) Representative: Roth, Andy Stefan
(86) International application number: PCT/EP2013/057759
(87) International publication number: WO 2013/156421

(56) References cited:
- US-A- 6 165 171
- US-A1- 2005 080 466
- US-A1- 2006 287 646
- US-A1- 2007 239 147
- US-A1- 2008 287 930
- US-A1- 2011 313 408
- US-B1- 6 428 532
- US-B1- 7 066 929

## Description

### TECHNICAL FIELD

The present specification relates to novel laser-based medical systems and methods, and more particularly to such systems and methods to treat deep surface tissue structures associated with with certain disorders using multiple laser beams.

### BACKGROUND ART

Laser / tissue interactions and therapeutic applications of laser light are two rapidly growing areas of research. As is well known to those skilled in the art, high intensity laser beams have the potential to destroy bodily tissue, a property that can be turned to useful purposes in the field of the treatment of certain disorders. Some of these disorders involve unwanted cell structures that can be found in deep surface tissues.

However, structures and tissues that are buried deep within healthy tissue pose a special problem for laser-based methods if used for a transcutaneous treatment. More particularly, as lasers are ultimately just coherent light sources, any attempt to "illuminate" a buried structure in the deep surface tissues with laser light must necessarily also illuminate the tissue that lies between the laser source at the surface and the target structure. Thus, the heat from a laser that is directed at a buried target has the potential to destroy not only the structure, but also to the tissue that encloses it, in particular the skin surface.

Therefore, the use of lasers in medicine or similar fields is limited by several factors; one is the penetration depth for controlled or significant energies. The overall limitation in getting to deeper transcutaneous targets (such as deep dermis, fat, tendons, etc., without burning the skin the same time), is the fact that the absorbed energy/temperature is reduced exponentially the deeper the laser radiation gets (Lambert-Beer's law). This means that most of the light energy is absorbed at the surface of the skin. The intensity reduces quickly the deeper the laser penetrates.

Furthermore, the transcutaneous treatment of deep surface tissue structures such as tumors can induce an excessive degree of trauma to the skin, such as a burning of the skin surface, leading to necrosis and scarring.

Some of the currently available methods and systems have attempted to overcome these negative effects by including a complex cooling system to cool down the skin, in an attempt to reduce excessive heat development at the surface of the skin and the resulting trauma to the epidermal layer of the skin. However, such cooling systems add complexity to implementation, often requires that laser power be increased, and also may not provide a desired or uniform level of cooling and trauma reduction in the skin. The combination of non-uniformity in cooling and increased laser power can put the skin at an even greater risk of damage. Also, adjusting the fluence (energy/area) delivered by a laser, as specified by current procedures, generally provides an inadequate level of control and often leads to either over-treatment or under-treatment. Over-treatment may cause scarring, and under-treatment may result in no observable improvement in the condition being treated.

US-A-5,746,738 discloses a medical system for the transcutaneous treatment of a target structure by laser light, comprising a laser beam delivery system producing a plurality of laser beams for simultaneously or alternately irradiating the target structure from multiple directions via the skin surface, wherein the laser beams individually are individually adjusted to have an energy level insufficient to ablate and/or burn the skin. However, the disclosed systems to not reduce or resolve the aforementioned issues.

US 7,066,929 B1 describes a medical system for performing selective photothermolysis of sub-cutaneous tissue. The medical system uses a plurality of beams of narrow band electromagnetic radiation, where each of the beams have energy that is insufficient to heat the tissue to a temperature which is high enough to destroy such tissue. To destroy the target tissue the individual beams are directed to overlap at the target so as to sufficient heat is generated inside the target tissue.

Furthermore, US 2011/0313408 A1 discloses a laser skin treatment process and system. The medical system comprises means for producing a first laser beam of long pulse duration and a second laser beam of short duration and a skin cooler for cooling the surface of a region of the skin. The main idea of the medical system described is that the system is designed to utilize the first laser beam for heating a volume of skin tissue below the cooled surface region to a temperature to produce skin tissue modification but below a skin tissue damage threshold. Within this heated region of tissue the second laser beam mechanically damages the target structure.

Therefore, effective and improved systems and methods that are able to transcutaneous treat unwanted structures in deep surface tissues are needed.

### DISCLOSURE OF INVENTION

The invention and its advantageous embodiments are defined in the appended set of claims. Methods mentioned hereinafter do not form part of the present invention.

In a first aspect, embodiments of the invention provide medical systems for the transcutaneous treatment of a deep tissue target structure by laser light. A laser beam delivery system produces a plurality of laser beams for simultaneously or alternately irradiating the target structure from multiple directions via the skin surface. Suitably, the laser beams are individually adjusted to have an energy level insufficient to ablate and/or burn the skin. The target structure comprises a deep tissue target structure. The laser beams have a combined energy level at their point of intersection to achieve a preselected thermal gradient sufficient to treat the deep tissue target structure. The medical system further comprises a cooling device for cooling the skin surface during irradiation. Furthermore, the laser beam delivery system comprises multiple lasers. The lasers 12, 14, 16, 18 are suitably mounted on axles 22, 24, 26, 28, and are rotatable by motors. The motors enables the angular orientation (θ; with respect to the vertical) of the lasers 12, 14, 16, 18 to be adjusted, so as to adjust the direction of the laser beam emanating therefrom.

The deep tissue target structure may be selected from the group consisting of collagen fibers, septal fibers, hair, blood vessels, nerves, glandular tissue of any type, superficial or deep fascia, nucleus pulposus, joints, cartilage, bone, water, fat, sweat glands, sebaceous glands, bacteria, varicose veins and tumor cells.

The deep tissue target structure may correlate with a disorder selected from the group consisting of cellulite, hyperhidrosis, cancer, backpain, acne, tumors, hypertrichosis, adipose tissue, skin irregularities, skin tightening and eye disorder.

The laser beam delivery system may comprise one laser that uses mirrors, rotating elements, scanners or other devices to direct the beams from different directions/angles towards the deep tissue target structure.

In embodiments, each laser beam may be continuous or pulsed.

The laser beams may be produced at the same time or alternated or repeated to achieve the preselected thermal gradient at the deep tissue target structure.

In still another aspect, embodiments of the invention are used in methods for the transcutaneous treatment of a deep tissue target structure in a patient comprising the steps of simultaneously or alternately irradiating the deep tissue target structure with a plurality of laser beams from multiple directions via the skin surface. The laser beams individually have an energy level insufficient to ablate and/or burn the skin. The target structure comprises a deep tissue target structure. The laser beams have a combined energy level at their point of intersection to achieve a preselected thermal gradient sufficient to treat the deep tissue target structure. The method further comprises cooling the skin surface during the irradiation.

The deep tissue target structure may be selected from the group consisting of collagen fibers, septal fibers, hair, blood vessels, nerves, glandular tissue of any type, superficial or deep fascia, nucleus pulposus, joints, cartilage, bone, water, fat, sweat glands, sebaceous glands, bacteria, varicose veins and tumor cells.

The deep tissue target structure may correlates with a disorder selected from the group consisting of cellulite, hyperhidrosis, cancer, backpain, acne, tumors, hypertrichosis, adipose tissue, skin irregularities, skin tightening and eye disorder.

Preferably, the plurality of laser beams are produced by multiple lasers.

In one embodiment, the plurality of laser beams are produced by one laser that uses mirrors, rotating elements, scanners or other devices to direct the beams from different directions/angles towards the deep tissue target structure.

In embodiments, each laser beam is continuous or pulsed.

The laser beams may be produced at the same time or alternated or repeated to achieve the selected thermal gradient at the deep tissue target structure.

In a further aspect, the system disclosed herein may be used for the treatment of a deep tissue target structure and/or for the treatment of a disorder selected from the group consisting of cellulite, hyperhidrosis, cancer, backpain, acne, tumors, hypertrichosis, adipose tissue, skin irregularities, skin tightening and eye disorder.

According to embodiments, the use may involve the deep tissue target structure being disrupted, ablated, coagulated, heated, emulgated and/or liquefied.

According to embodiments, the deep tissue target structure is selected from the group consisting of collagen fibers, septal fibers, hair, blood vessels, nerves, glandular tissue of any type, superficial or deep fascia, nucleus pulposus, joints, cartilage, bone, water, fat, sweat glands, sebaceous glands, bacteria, varicose veins and tumor cells.

The technology disclosed herein relates to a medical system, and corresponding methods and uses, comprising the novel use of lasers to treat tissue. An advantage of the present invention is that this is done in a manner so as to transfer a desired amounts of energy to deeper tissues, such as deep dermis, fat, deep vessels, fascia, tumors and others, without burning the skin, in particular the skin surface.

A laser focusing system for transcutaneous treatment has been developed that allows for a plurality of laser beams to intersect at a single focal point within a patient. A further advantage of the invention is that, collectively, the beams have enough energy to treat tissue or unwanted structures in the tissue at the focal point without burning and/or ablating the skin, in particular the skin surface.

A further advantage of the invention is that each individual laser beam will not by itself damage tissue, in particular the skin surface tissue. The use of several relatively low-powered lasers that treat tissue only when collectively directed to a single target has inherent safety features. For example, a single laser having a high energy level could not be used for the treatment of deep surface target structures since it would burn the upper layers of the skin by the high thermal gradient caused by the high energy level.

A further advantage of the invention is that it avoids or substantially reduces the prior limitation - in getting to deeper transcutaneous targets, such as deep dermis, fat, tendons, etc., due the fact that the absorbed energy/temperature is reduced exponentially the deeper the laser gets (Lambert-Beer's law)-without burning the skin the same time.

### BRIEF DESCRIPTION OF THE DRAWINGS

FIG. 1 illustrates schematically a medical system for the transcutaneous treatment of tissue, according to an embodiment of the invention;
FIG. 2 shows the function of the medical system of FIG. 1, in use;
FIG. 3 (PRIOR ART) is a diagram showing the effect of the use of a traditional laser system in correlation with the penetration depth, for transcutaneous treatment; and
FIG. 4 is a diagram illustrating the effect of the use of a medical laser system according to an embodiment of the present invention, in correlation with the penetration depth, for transcutaneous treatment.

### DETAILED DESCRIPTION OF THE PREFERRED EMBODIMENTS

Unless indicated otherwise herein, individual system design features, combinations of components, method steps or other techniques disclosed herein may be combined, in accordance with embodiments of the present invention, with any or all other system design features, combinations of components, method steps or other techniques disclosed herein.

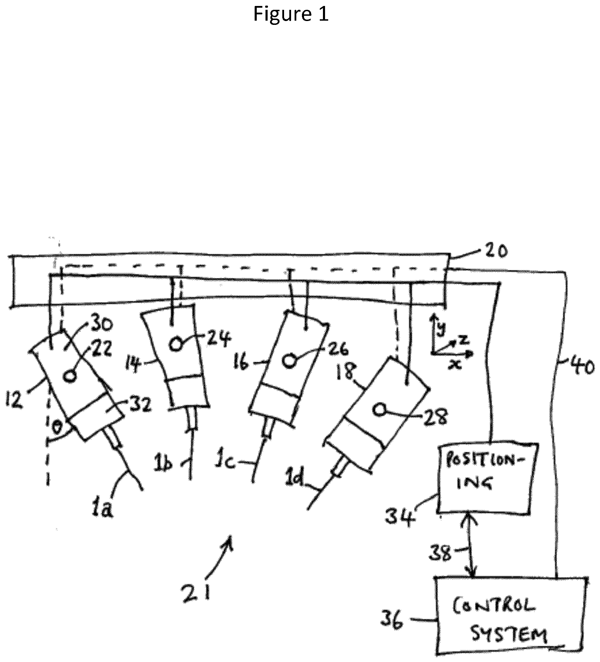

FIG. 1 illustrates schematically a medical system 10 for the transcutaneous treatment of tissue, according to an embodiment of the invention.

A plurality (here 4) of laser devices 12, 14, 16, 18 are mounted (e.g. above a patient for treatment thereof), on a housing or frame schematically illustrated at 20. Collectively, hereinafter these are referred to as a laser beam delivery system 21.

As also indicated in relation to laser device 12, this comprises a laser source 30 and focusing optics 32. The construction of the other laser devices is preferably the same, but may be different to suit different treatments.

The laser devices 12, 14, 16, 18 emit respectively laser beams 1a, 1b, 1c, 1d, for treatment of tissue, as discussed below.

In accordance with embodiments of the invention, a variety of types of lasers 12, 14, 16, 18 may be used, e.g., He:Ne, Nd:YAG, Er:YAG and others. The power level, the pulse duration, the frequency of the pulses, and the distance from the tip of the laser 12, 14, 16, 18 to the point where the laser beams converge, can be varied.

The laser beam delivery system 21 comprises multiple lasers 12, 14, 16, 18 providing the plurality of laser beams 1a, 1b, 1c, 1d; and there may be two, three, four or more beams. According to embodiments of the present invention, each laser beam 1a, 1b, 1c, 1d produced by the laser beam delivery system 21 may be continuous or pulsed.

In one embodiment, the laser beams 1a, 1b, 1c, 1d are produced at the same time, in another embodiment the laser beams 1a, 1b, 1c, 1d are produced alternated or repeated to achieve the preselected thermal gradient at the deep tissue target structure. Alternatively, the laser beam delivery system 21 may include two or more different types of laser light sources 30 to provide a variety of different wavelengths or wavelength ranges. Optical beams 1a, 1b, 1c, 1d from different laser light sources 30 may be directed to the targeted structure on a one-by-one basis or at the same time.

As mentioned above, examples of laser light sources 30 include, but are not limited to, diode lasers, diode- pumped solid state lasers, Er : YAG lasers, Nd : YAG lasers, argon-ion lasers, He-Ne lasers, carbon dioxide lasers, eximer lasers, ruby lasers, and so forth. For certain embodiments, a laser light source 30 is desirably a diode laser, such as an infrared diode laser. However, it should be recognized that the selection of a particular type of laser light source 30 in the laser beam delivery system is dependent on the types of the target structure and the type of the deep surface tissue to be treated using the medical system according to the present invention. The laser beam delivery system 21 may include one particular type of laser light source 30 capable of providing one wavelength or wavelength range.

The lasers 12, 14, 16, 18 are suitably mounted on axles 22, 24, 26, 28, and are rotatable by motors (not shown), e.g. stepper motors. As indicated in relation to laser 12, operation of the motors enables the angular orientation (θ; with respect to the vertical) of the laser 12 to be adjusted, so as to adjust the direction of the laser beam 1a emanating therefrom. Adjustment of the other lasers 14, 16, 18 is achieved similarly.

The spatial position of the frame 20, and therefore of the lasers 12, 14, 16, 18 is adjusted by positioning mechanism 34, which is schematically illustrated for the present purposes. It will be appreciated by persons skilled in the art that positioning mechanism 34 may comprise a number of arms, links, axles, hinges, motors, gears and the like, in order to move the frame through space for optimal positioning of the lasers 12, 14, 16, 18 in relation to the tissue (not shown) to be treated. In particular, the positioning mechanism 34 is at least adapted for moving the frame 20 for vertical positioning in the y direction in Fig. 1, but is preferably adapted to move in the x, y and z directions.

The operation of the system 10 is controlled by processor-based control system 36, which suitably includes user input and graphical user interface devices (not shown) to assist a practitioner in deploying treatment. In use, the control system 36 sends control signals to and receives (position) signals from positioning mechanism 34 via lines 38. In addition, control system 36 sends control signals to lasers 12, 14, 16, 18 via lines 40.

In embodiments, the control system 36 is electronically coupled to the laser beam delivery system 21 via any wire or wireless transmission channel and functions to control operation of the laser beam delivery system 21, including the laser sources 30, focusing element, or both. By way of example, the control system 36 can activate one or more laser light sources 30 of the laser beam delivery system as well as control a variety of optical parameters associated with an activated laser light source 30. As another example, the control system 36 can control focusing elements 32 to control or adjust a pattern of optical energy that is directed to the targeted structure.

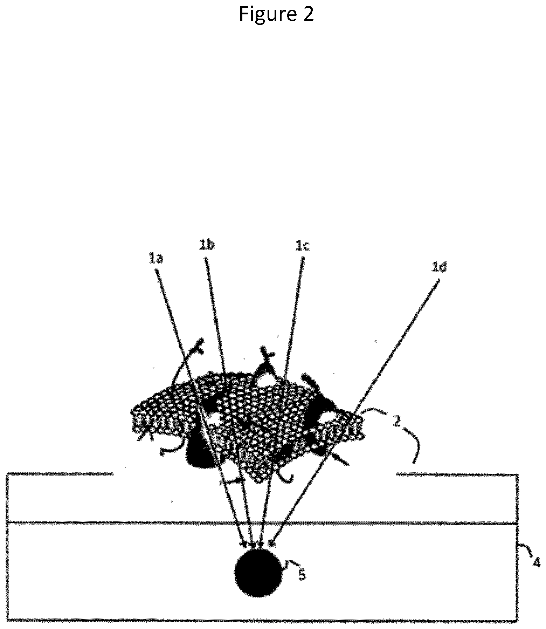

FIG.2 shows the principle of the medical system of FIG. 1, in use. Each of the laser beams (1a, 1b, 1c, 1d) is delivering low energy irradiation to the target structure 5 in a deep tissue 4 via the skin surface 2. Examples of the deep tissue target structure 5 are collagen fibers, septal fibers, hair, blood vessels, nerves, glandular tissue of any type, superficial or deep fascia, nucleus pulposus, joints, cartilage, bone, water, fat, sweat glands, sebaceous glands, bacteria, varicose veins and tumor cells.

Examples for disorders correlating with the unwanted deep tissue target structures are cellulite, hyperhidrosis, cancer, backpain, acne, tumors, hypertrichosis, adipose tissue, skin irregularities, skin tightening and eye disorder.

To protect the skin surface 2 and to reach even deeper targets 5 the skin surface 2 is cooled by contact cooling (e.g. using a heat exchanger or heat sink) or the use of cryogenic or other techniques; in particular, the skin surface 2 make be cooled with a cooling device such as a contact pat or a cryogen spray (in each case, not shown).

Collectively, the beams 1a, 1b, 1c, 1d have enough energy to treat the deep tissue target structure 5 at their focal point (intersection). However, each individual laser beam 1a, 1b, 1c, 1d will not by itself damage tissue above the intersection of the laser beams 1a, 1b, 1c, 1d. The use of several relatively low-powered lasers 12-18 to treat deep surface tissue only when collectively directed to a single target 5 has inherent safety features.

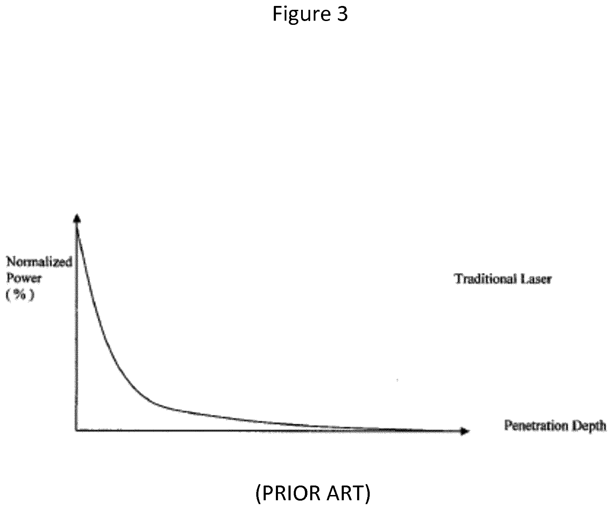

FIG. 3 (PRIOR ART) is a diagram showing the effect of the use of a traditional laser system in correlation with the penetration depth for transcutaneous treatment. The normalized power value of radiation incident upon tissue falls away exponentially with depth of penetration into the tissue.

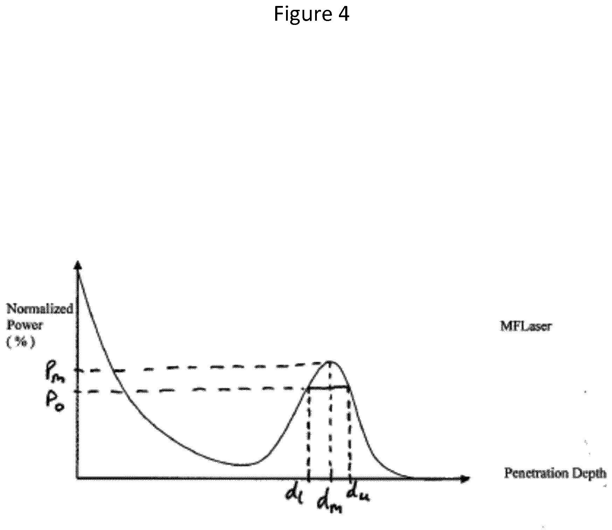

FIG. 4 is a diagram illustrating the effect of the use of a medical laser system according to the present embodiment of the invention in correlation with the penetration depth for transcutaneous treatment. The effect of the use of multiple lasers focused upon the same point/area is that power is maximum (P_{M}) at depth dₘ, whereas, for example, a usable operational range may be available between dₗ and dᵤ, corresponding to power Pₒ. The control system 36 may operate in use to keep the laser beam delivery system 21 operating between Po and P_{M}, and/or to adjust dₗ, dₘ and dᵤ. Preferably, the depth range dₗ to dᵤ lies within the depth range of the target structure 5 at that (x, z) location.

Due to the cooling of the skin surface for example by a cooling device such as a contact cooling pat or a cryogen spray, the overlaying tissue like the skin surface is not burned and/or ablated. In an advantageous embodiment, no deeper than a second degree burn is produced on the skin, in particular on the skin surface, and preferably no deeper than a first degree burn.

In advantageous embodiments the medical system 10 according to the present disclosure comprises a laser beam delivery system 21 for producing multiple deep laser beams 1a, 1b, 1c, 1d that are directed from multiple directions through the skin 2 towards the target 5 like a deep surface tissue or a deep surface tissue structure. In an advantageous embodiment, the laser beams 1a, 1b, 1c, 1d are focused laser beams.

Surprisingly it was found that the energies the beams 1a, 1b, 1c, 1d adds up to a higher overall energy, to achieve the desired aim of treating the target 5, reach a thermal gradient, to disrupt, ablate, coagulate, heat, emulgate, liquefy, etc.; but due to the cooling and the use of a plurality of laser beams 1a, 1b, 1c, 1d, the upper skin 2 is not damaged (burned or ablated). According to embodiments of the present invention, the cooling of the skin surface 2 allows the practitioner to treat very deep surface tissue structures 5, as higher energy laser beams 1a, 1b, 1c, 1d may be used.

Further embodiments of the present disclosure pertains to methods for the transcutaneous treatment of a deep tissue target structure in a patient comprising the steps of simultaneously or alternately irradiating the deep tissue target structure with a plurality of laser beams from multiple directions via the skin surface, wherein the laser beams individually have an energy level insufficient to ablate and/or burn the skin, and the laser beams have a combined energy level at the point of intersection to achieve a thermal gradient sufficient to treat the deep tissue target structure, and wherein the skin surface is cooled during the irradiation.

Furthermore, some embodiments relates to uses of the medical system according to the present disclosure for the treatment of a deep tissue target structure.

In accordance with the methods and uses, by selecting the appropriate laser wave length, wattage, pulse rate, the distance from each laser source 30 to the point of intersection, and the number of lasers 12-18 , the effect on body tissue can be carefully and precisely controlled.

The following example is offered for illustrative purpose only, and is not intended to limit the scope of the present disclosure in any way.

### Examples

In the following, an example of the present disclosure is provided showing the improved treatment of deep-surface target structures 5 when using a medical system 10 according to the present invention. It should be understood that this example is for illustrative purpose only and is not to be construed as limiting this disclosure in any manner.

### Example 1:

Three 1064nm long pulse lasers 12-16 were implemented in a treatment of fresh cadaver tissue. Subclinical fluence settings were used.

A thermometer was placed at 3mm depth, and in a second study in 5mm depth. Firing of a single 1064nm laser with a subclinical fluence did not raise the temperature at 3mm depth significantly. At 5mm depth, no temperature change could be observed.

Firing the three 1064nm lasers 12-16 from different angles, using surface cooling, and aiming all beams 1a-1c at one spot at 3mm depth produced a significant observable rise in the temperature at that spot.

Histology with Azan staining (see Mallory FB (1900) A contribution to staining methods. Journal of Experimental Medicine 5: 15-20*)* of that area showed no damage to superficial structures such as epidermis or superficial dermis. In the deep dermis - approx. 3mm deep - denatured collagen could be seen.

In another procedure, the three beams 1a-1c were aimed at 5mm depth. The same laser energies were fired; and a significant change of the temperature was observed.

Histology did not show significant changes.

Further embodiments of the invention will be apparent to persons skilled in the art.

In an advantageous embodiment, the medical system 10 includes an optical delivery system such as a laser beam delivery system, which includes an optical source, in particular a laser or a laser diode. The optical source functions to provide optical energy that can be directed to a deep surface target structure, such as a tumor. In some embodiments, the optical source provides optical energy in the form of one or more optical beams, in particular laser beams, which can be pulsed or continuous wave and coherent or incoherent.

In certain embodiments, the optical source may be implemented, at least in part, using one or more light sources, such as laser light sources. For certain applications, the optical source desirably includes multiple laser light sources, which can be arranged in an array, such as a one-dimensional array or a two-dimensional array. A laser light source can provide one or more optical beams having particular optical parameters, such as optical fluence, power, timing, pulse duration, inter-pulse duration, wavelength (s), and so forth, to produce a desired treatment effect for the targeted structure. By way of example, a laser light source can provide an optical beam having a wavelength or range of wavelengths between approximately 200 nm and 20,000 nm, such as between approximately 600 nm and 4000 nm.

In an advantageous embodiment, the laser beam delivery system may also include a focusing element that is optically coupled to the laser(s). The focusing element functions to direct optical energy from the laser to the targeted structure. In one embodiment, the focusing element directs optical energy to the targeted structure by focusing the power of the optical energy to one or more deep surface tissue target structure.

Use of a pattern of optical energy also can facilitate multiple treatments that may be needed to produce a full desired effect by allowing an individual treatment to be milder and with lower risk to a patient. Furthermore, visible impressions of treatment can be reduced by using a pattern of treatment where an individual treatment zone is on the same or smaller scale than the normal visible texture or constituents of the skin itself.

The present invention has now been described in accordance with several exemplary embodiments, which are intended to be illustrative in all aspects, rather than restrictive. Thus, the present invention is capable of many variations in detailed implementation, which may be derived from the description contained herein by a person of ordinary skill in the art. All such variations are considered to be within the scope of the present invention as defined by the following claims and their legal equivalents.

## Claims

1. A medical system (10) for the transcutaneous treatment of a target structure (5 with a deep tissue target structure by laser light, comprising a laser beam delivery system (21) producing a plurality of laser beams (1a-1d) for simultaneously or alternately irradiating the target structure (5) from multiple directions via the skin surface (2), wherein the laser beams (1a-1d) are individually adjusted to have an energy level insufficient to ablate and/or burn the skin (2); wherein the laser beams (1a-1d) have a combined energy level at their point of intersection, which is sufficient to disrupt, ablate, coagulate or liquefy tissue, and in that the medical system (10) further comprises a cooling device for cooling the skin surface (2) during irradiation, whereupon a control system (36) is electronically coupled to the laser beam delivery system (21) via any wire or wireless transmission channel and functions to control operation of the laser beam delivery system (21) as well as to control a variety of optical parameters associated with an activated laser light source (30) of the laser beam delivery systems (21), whereupon the laser beam delivery system comprises multiple lasers (12,14,16,18) providing the plurality of laser beams (1a-1d), **characterized in that** the lasers (12, 14, 16, 18) are mounted on respective axles (22, 24, 26, 28) and are rotatable by motors so as to adjust the angular orientation of the respective laser and the direction of the laser beam emanating therefrom.

2. The medical system (10) according to claim 1, wherein the laser beams (1a-1d) have a combined energy level at their point of intersection to achieve a preselected thermal gradient sufficient to treat the deep tissue target structure (5) selected from the group consisting of collagen fibers, septal fibers, hair, blood vessels, nerves, glandular tissue of any type, superficial or deep fascia, nucleus pulposus, joints, cartilage, bone, water, fat, sweat glands, sebaceous glands, bacteria, varicose veins and tumor cells.

3. The medical system (10) according to any one of claims 1 to 2, wherein the laser beams (1a-1d) have a combined energy level at their point of intersection to achieve a preselected thermal gradient sufficient to treat the deep tissue target structure (5) which correlates with a disorder selected from the group consisting of cellulite, hyperhidrosis, cancer, backpain, acne, tumors, hypertrichosis, adipose tissue, skin irregularities, skin tightening and eye disorder.

4. The medical system (10) according to any one of claims 1 to 3, wherein the laser beam delivery system (21) comprises one laser that uses mirrors, rotating elements, scanners or other devices to direct the beams from different directions/angles towards the deep tissue target structure (5).

5. The medical system (10) according to any one of claims 1 to 4, wherein each laser beam (1a-1d) is continuous or pulsed.

6. The medical system according to any one of claims 1 to 5, wherein the laser beams (1a-1d) are produced at the same time or alternated or repeated to achieve the preselected thermal gradient at the deep tissue target structure (5).

## Patentansprüche

1. Ein medizinisches System (10) zur transkutanen Behandlung einer Zielstruktur (5) mit einer Tiefengewebestruktur mittels Laserlicht, umfassend ein Laserstrahlliefersystem (21), das eine Vielzahl von Laserstrahlen (1a-1d) produziert, zur simultanen oder abwechselnden Bestrahlung der Zielstruktur (5) von mehreren Richtungen durch die Hautoberfläche (2), wobei die Laserstrahlen (1a-1d) individuell so eingestellt sind, dass sie ein Energielevel haben welches nicht ausreicht die Haut (2) abzutragen und/oder zu verbrennen, wobei die Laserstrahlen (1a-1d) ein kombiniertes Energielevel am Punkt ihrer Überschneidung haben, das ausreichend ist um Gewebe zu zerstören, abzutragen, koagulieren oder zu verflüssigen, und dass das medizinische System (10) ferner eine Kühlvorrichtung zu Kühlung der Hautoberfläche (2) während der Bestrahlung umfasst, wobei ein Kontrollsystem (36) elektronisch mit dem Laserstrahlliefersystem (21) mittels einem Kabel oder einem drahtlosen Übertragungskanal gekoppelt ist, um den Betrieb des Laserstrahlliefersystems (21) wie auch eine Vielfalt von optischen Parametern, die mit einer aktivierten Laserlichtquelle (30) des Laserstrahlliefersystems (21) in Verbindung stehen, zu kontrollieren, wobei das Laserstrahlliefersystems mehrere Laser (12, 14, 16, 18) umfasst, die die Vielzahl von Laserstrahlen (1a-1d) liefern, **dadurch gekennzeichnet, dass** die Laser (12, 14, 16, 18) auf entsprechenden Achsen (22, 24, 26, 28) montiert sind und mittels Motoren rotierbar sind, um so die Winkelorientierung des entsprechenden Lasers und die Richtung der daraus ausgehenden Laserstrahlen einzustellen.

2. Das medizinische System (10) gemäß Anspruch 1, wobei die Laserstrahlen (1a-1d) ein kombiniertes Energielevel am Punkt ihrer Überschneidung haben, um einen vorgewählten thermischen Gradienten zu erreichen, welcher ausreicht die Tiefengewebestruktur (5) ausgewählt aus der Gruppe bestehend aus Kollagenfasern, septalen Fasern, Haaren, Blutgefäßen, Nerven, Drüsengewebe jeglicher Art, oberflächliche oder tiefen Faszien, Gallertkerne, Gelenke, Knorpel, Knochen, Wasser, Fett, Schweißdrüsen, Talgdrüsen, Bakterien, Krampfadern und Tumorzellen, zu behandeln.

3. Das medizinische System (10) gemäß einem der Ansprüche 1 bis 2, wobei die Laserstrahlen (1a-1d) ein kombiniertes Energielevel am Punkt ihrer Überschneidung haben um einen vorgewählten thermischen Gradienten zu erreichen, der ausreicht die Tiefengewebestruktur (5), die mit einer Krankheit korreliert ausgewählt aus der Gruppe bestehend aus Zellulite, Hyperhidrosis, Krebs, Rückenschmerzen, Akne, Tumore, Hypertrichose, Fettgewebe, Hautunregelmäßigkeiten, Hautstraffung und Augenkrankheiten, zu behandeln.

4. Das medizinische System (10) gemäß einem der Ansprüche 1 bis 3, wobei das Laserstrahlliefersystem (21) einen Laser umfasst, der Spiegel, Drehvorrichtungen oder Scanner oder andere Vorrichtungen verwendet, um die Strahlen aus unterschiedlichen Richtungen/Winkeln zu der Tiefengewebestruktur (5) zu lenken.

5. Das medizinische System (10) gemäß einem der Ansprüche 1 bis 4, wobei jeder Laserstrahl (1a-1d) kontinuierlich oder gepulst ist.

6. Das medizinische System (10) gemäß einem der Ansprüche 1 bis 5, wobei die Laserstrahlen (1a-1d) zur selben Zeit oder abwechselnd oder wiederholt produziert werden, um den vorgewählten thermischen Gradienten bei der Tiefengewebestruktur (5) zu erreichen.

## Revendications

1. Système médical (10) pour le traitement transcutané d'une structure cible (5) avec une structure cible de tissu profond par une lumière laser, comprenant un système de distribution de faisceau laser (21) produisant une pluralité de faisceaux lasers (1a à 1d) pour irradier simultanément ou alternativement la structure cible (5) à partir de multiples directions via la surface de peau (2), dans lequel les faisceaux lasers (1a à 1d) sont ajustés individuellement pour avoir un niveau d'énergie insuffisant pour pratiquer une ablation de et/ou brûler la peau (2) ; dans lequel les faisceaux lasers (1a à 1d) ont un niveau d'énergie combiné au niveau de leur point d'intersection qui est suffisant pour interrompre, pratiquer une ablation, coaguler ou liquéfier un tissu, et en ce que le système médical (10) comprend en outre un dispositif de refroidissement pour refroidir la surface de peau (2) pendant l'irradiation, sur quoi un système de contrôle (36) est couplé électroniquement au système de distribution de faisceau laser (21) via un quelconque canal de transmission filaire ou sans fil et fonctionne pour contrôler un fonctionnement du système de distribution de faisceau laser (21) ainsi que pour contrôler une variété de paramètres optiques associés à une source de lumière laser (30) activée du système de distribution de faisceau laser (21), sur quoi le système de distribution de faisceau laser comprend de multiples lasers (12, 14, 16, 18) fournissant la pluralité de faisceaux lasers (1a à Id),
**caractérisé en ce que** les lasers (12, 14, 16, 18) sont montés sur des essieux (22, 24, 26, 28) respectifs et peuvent être mis en rotation par des moteurs de manière à ajuster l'orientation angulaire du laser respectif et la direction du faisceau laser émanant de celui-ci.

2. Système médical (10) selon la revendication 1, dans lequel les faisceaux lasers (1a à 1d) ont un niveau d'énergie combiné au niveau de leur point d'intersection pour atteindre un gradient thermique présélectionné suffisant pour traiter la structure cible de tissu profond (5) sélectionnée dans le groupe consistant en des fibres collagènes, des fibres septales, des cheveux, des vaisseaux sanguins, des nerfs, un tissu glandulaire d'un quelconque type, un fascia superficiel ou profond, un noyau gélatineux, des articulations, un cartilage, un os, de l'eau, de la graisse, des glandes sudoripares, des glandes sébacées, des bactéries, des varices et des cellules tumorales.

3. Système médical (10) selon l'une quelconque des revendications 1 à 2, dans lequel les faisceaux lasers (1a à 1d) ont un niveau d'énergie combiné au niveau de leur point d'intersection pour atteindre un gradient thermique présélectionné suffisant pour traiter la structure cible de tissu profond (5) qui correspond à un trouble sélectionné dans le groupe consistant en de la cellulite, une hyperidrose, un cancer, un mal de dos, de l'acné, des tumeurs, une hypertrichose, un tissu adipeux, des irrégularités de la peau, un resserrement de la peau et un trouble oculaire.

4. Système médical (10) selon l'une quelconque des revendications 1 à 3, dans lequel le système de distribution de faisceau laser (21) comprend un laser qui utilise des miroirs, des éléments rotatifs, des scanners ou d'autres dispositifs pour diriger les faisceaux à partir de différentes directions/différents angles vers la structure cible de tissu profond (5).

5. Système médical (10) selon l'une quelconque des revendications 1 à 4, dans lequel chaque faisceau laser (1a à 1d) est continu ou pulsé.

6. Système médical selon l'une quelconque des revendications 1 à 5, dans lequel les faisceaux lasers (1a à 1d) sont produits simultanément ou alternés ou répétés pour atteindre le gradient thermique présélectionné au niveau de la structure cible de tissu profond (5).
